(19) Europäisches Patentamt
European Patent Office
Office européen des brevets

(11) Publication number: 0 032 048
A1

(12) EUROPEAN PATENT APPLICATION

(21) Application number: 80304709.1

(22) Date of filing: 23.12.80

(51) Int. Cl.³: C 07 D 207/333
C 07 D 207/337, A 61 K 31/40

(30) Priority: 08.01.80 GB 8000508

(43) Date of publication of application: 15.07.81 Bulletin 81/28

(84) Designated Contracting States: BE CH DE FR GB LI NL

(71) Applicant: BEECHAM GROUP LIMITED
Beecham House Great West Road
Brentford, Middlesex(GB)

(72) Inventor: Goudie, Alexander Crossan
72 North Brooks
Harlow Essex(GB)

(72) Inventor: Ward, Robert William
332 Willowfield Tower
Harlow Essex(GB)

(74) Representative: Dawson, Hugh Bainforde, Dr. et al,
European Patent Attorney BEECHAM PHARMACEUTICALS Yew Tree Bottom Road
Epsom, Surrey KT18 5XQ.(GB)

(54) Pyrroloylpyrrole derivatives, processes for their preparation and their use.

(57) Compounds of formula (I):

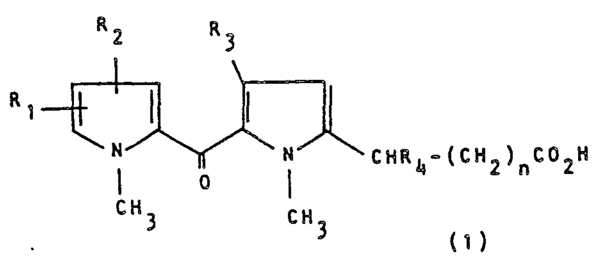

(1)

and pharmaceutically acceptable salts and pro-drugs thereof, wherein:

$R_1$ and $R_2$ are the same or different and are hydrogen, halogen or $C_{1-4}$ alkyl;

$R_3$ is hydrogen or $C_{1-6}$ alkyl

$R_4$ is hydrogen or methyl; and

n is 0 or 1 having useful pharmacological activity, processes for their preparation and their use

EP 0 032 048 A1

COMPLETE DOCUMENT

# Pyrroloylpyrrole derivatives, processes for their preparation and their use

The present invention relates to cyclic compounds having useful pharmacological activity, to processes for their preparation and to pharmaceutical compositions containing them.

Tolmetin, a clinically used anti-inflammatory and analgesic agent of the formula (A):

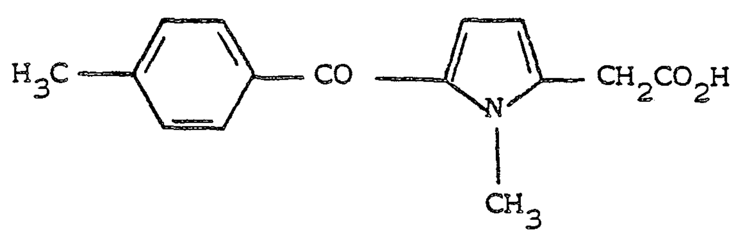

(A)

and related compound have been described in J. Pharmacol. Exptl. Therap. 1973, 185, 127-138, U.S. Patent Specification No. 3,752,826 and U.K. Patent Specification No. 1,195,628. It has now been found that certain other arylacetic acids possess good anti-inflammatory and analgesic activity.

Accordingly, the present invention provides compounds of the formula (I):

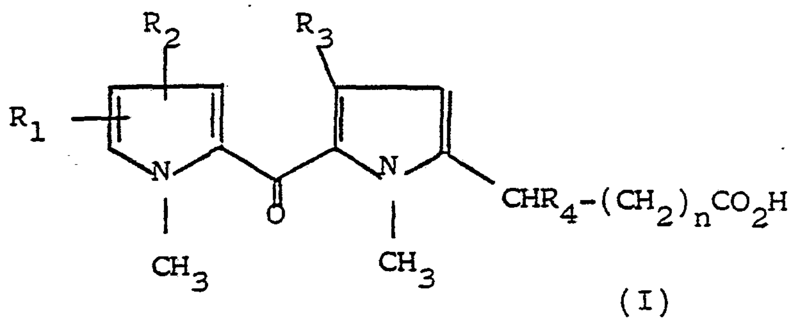

(I)

and pharmaceutically acceptable salts and pro-drugs thereof, wherein:

$R_1$ and $R_2$ are the same or different and are hydrogen, halogen or $C_{1-4}$ alkyl;

$R_3$ is hydrogen or $C_{1-6}$ alkyl

$R_4$ is hydrogen or methyl; and

n is 0 or 1.

When used herein the term "pro-drug" means a compound metabolised in vivo to a compound of the formula (I) or its salt. A pro-drug may be identified by administering the pro-drug to a mammal such as a rat, mouse, dog, monkey or man and identifying the compound of the formula (I) or its salt, in for example blood or urine.

One class of pro-drugs of the compounds of the formula (I) consists of in vivo hydrolysable esters. Such esters may be simple alkyl esters such as the methyl, ethyl, propyl or butyl esters, simply substituted alkyl esters such as the methoxymethyl, 2-methoxyethyl, 2-hydroxyethyl or benzyl esters or other esters conventionally used in the medical arts as pro-drugs such as a $C_{1-4}$ acyloxymethyl, $\alpha$- $C_{1-4}$ acyloxyethyl, $C_{1-4}$

alkoxycarbonyloxymethyl, α-$C_{1-4}$ alkoxycarbonyloxymethyl, or phthalidyl.

A further class of pro-drugs for the compounds of the formula (I) consists of in vivo hydrolysable amides thereof such as the primary amide, lower alkylamides and di-lower alkylamides thereof.

Another class of pro-drugs for the compound of the formula (I) consists of the analogous compounds of lower oxidation state, namely the corresponding compounds in which the $CO_2H$ groups is replaced by a CHO or $CH_2OH$ group.

A particularly suitable class of pro-drugs is that consisting of corresponding compounds wherein the $CO_2H$ group is replaced by a group of one of the subformulae (a) - (j):

| | |
|---|---|
| $-CH_2-CO-CH_3$ | (a) |
| $-CH_2-CHOH-CH_3$ | (b) |
| $-CH=C(OR_5)-CH_3$ | (c) |
| $-CHOH-CO-CH_3$ | (d) |
| $-CHOH-CHOH-CH_3$ | (e) |
| $-CH_2-C(OR_5)=CH_2$ | (f) |
| $-CH_2-C(OCOR_5)=CH_2$ | (g) |
| $-CH=C(OCOR_5)-CH_3$ | (h) |
| $-CH_2-C(OR_6)(OR_7)-CH_3$ | (i) |
| $-CH_2-CH(OCOR_8)-CH_3$ | (j) |

wherein:

$R_5$ is $C_{1-4}$ alkyl;

$R_6$ and $R_7$ are each $C_{1-4}$ alkyl or together are $-CH_2CH_2-$ or $-CH_2CH_2CH_2-$; and

$R_8$ is $C_{1-4}$ alkyl optionally substituted by phenyl

or optionally salified amino; $R_8$ is preferably methyl.

Particularly suitable $C_{1-4}$ alkyl groups are methyl or ethyl.

Particularly suitable pro-drugs for the compound of the present invention include those containing a group of the sub-formulae (a), (b), (d) or (e) as hereinbefore defined, that is, compounds of the formulae (II), (III), (IV) or (V).

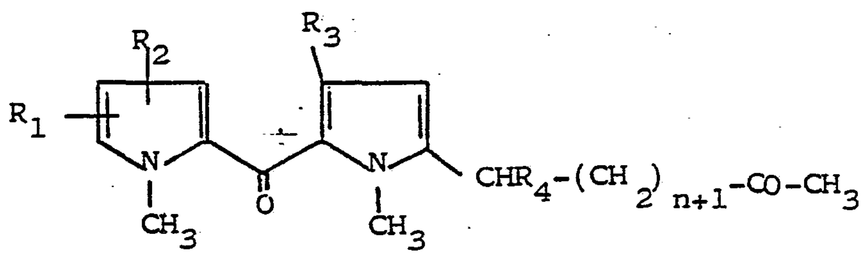

(II)

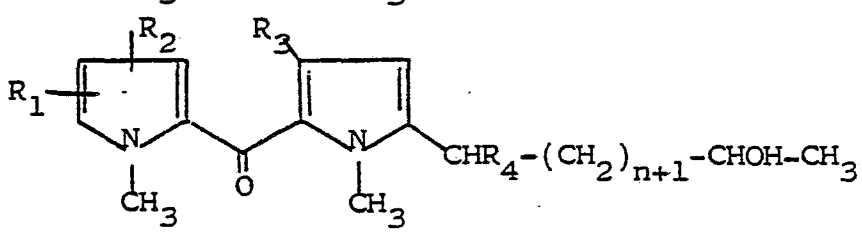

(III)

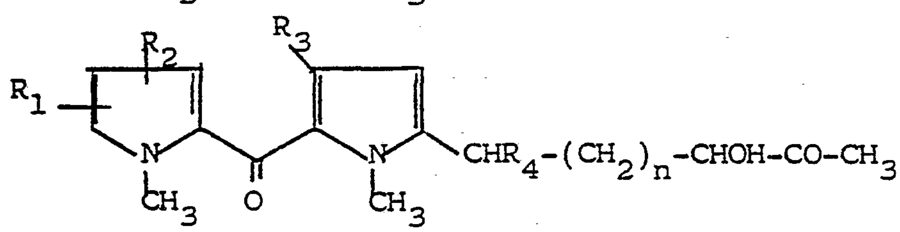

(IV)

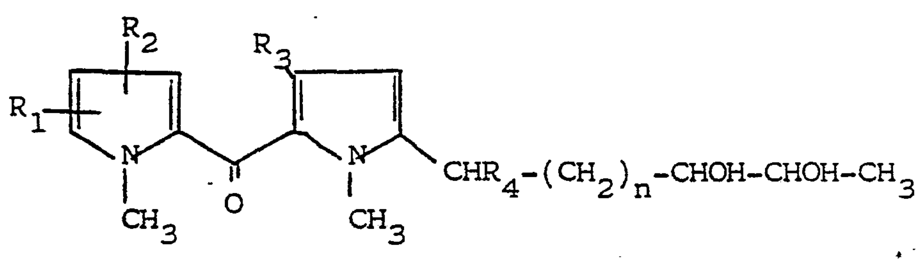

(V)

Certain preferred pro-drugs are those containing a group of the sub-formula (a), that is, compounds of the formula (II).

Apt pharmaceutically acceptable salts for the compounds of the formula (I) include alkali metal and alkaline earth metal salts, particularly the sodium, potassium, calcium and magnesium salts, and salts of pharmaceutically acceptable nitrogenous bases such as the ammonium salt and salts of protonated pharmaceutically acceptable amino acids, such as $H_3^+N.CH_2.COOH$.

In the compounds of the formula (I) and the pharmaceutically acceptable salts and pro-drugs thereof $R_1$ and $R_2$ may be the same or different and may be hydrogen, halogen or $C_{1-4}$ alkyl.

When $R_1$ or $R_2$ is halogen it is usually chlorine or bromine. When $R_1$ and $R_2$ are halogen they will usually be the same.

When one of $R_1$ and $R_2$ is hydrogen and the other is halogen, the halogen atom is often in the 4'-position.

When $R_1$ and $R_2$ are both halogen, they are often in the 4'- and 5'-positions.

When $R_1$ or $R_2$ is $C_{1-4}$ alkyl, suitable examples thereof include methyl, ethyl and n- and iso-propyl, more suitably methyl. When $R_1$ and $R_2$ are both $C_{1-4}$ alkyl they are usually the same.

When one of $R_1$ and $R_2$ is hydrogen and the other is $C_{1-4}$ alkyl, the alkyl group is often in the 5'-position.

When both $R_1$ and $R_2$ are $C_{1-4}$ alkyl, they are often in the 3'- and 5'-positions or the 4'- and 5'-positions.

In the compounds of the formula (I), certain specific moieties of the formula:

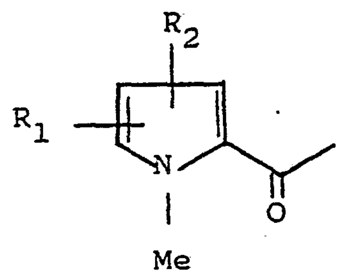

are:

4-chloropyrroloyl, 4-bromopyrroloyl, 4,5-dichloropyrroloyl, 4,5-dibromopyrroloyl, 5-methylpyrroloyl, 5-ethylpyrroloyl, 3,5-dimethylpyrroloyl, 3,5-diethylpyrroloyl, 4,5-dimethylpyrroloyl, 4,5-diethylpyrroloyl.

$R_1$ and $R_2$ will preferably both be hydrogen.

$R_3$ may be hydrogen or $C_{1-4}$ alkyl. When $R_3$ is $C_{1-4}$ alkyl, suitably examples thereof include methyl, ethyl and n- and iso-propyl, more suitably methyl. $R_3$ is preferably methyl.

$R_4$ is preferably hydrogen.

n is preferably 0.

From the foregoing it will be realised that certain particularly suitable compounds of invention are those of the formula (VI):

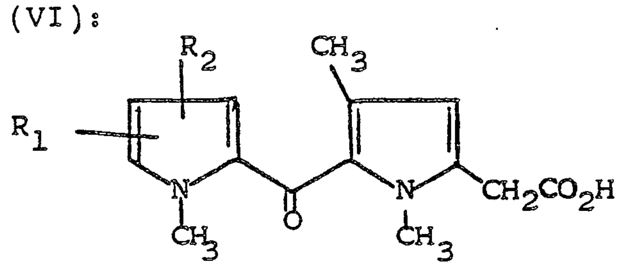

(VI)

wherein the $R_1$ and $R_2$ are as defined in formula (I), pharmaceutically acceptable salts thereof and pro-drugs thereof of the formulae (VIII), (IX), (X), (XI) and (XII):

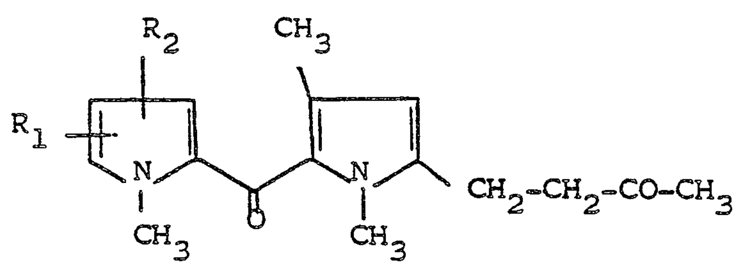

(VII)

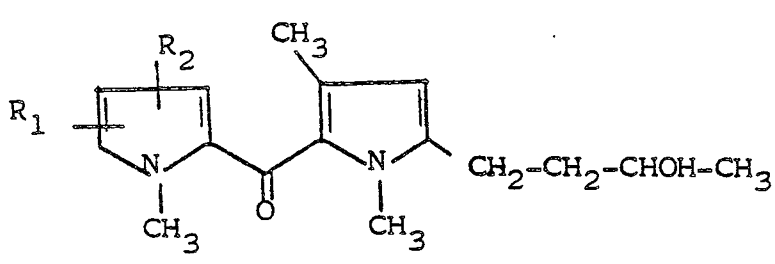

(VIII)

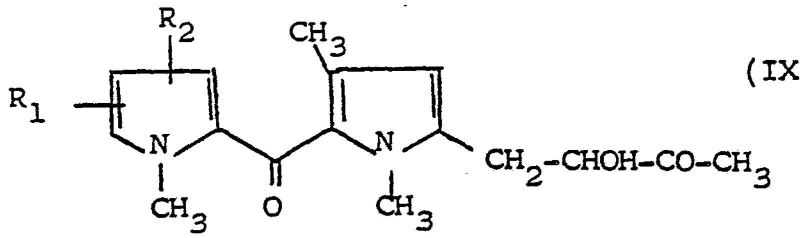

(IX)

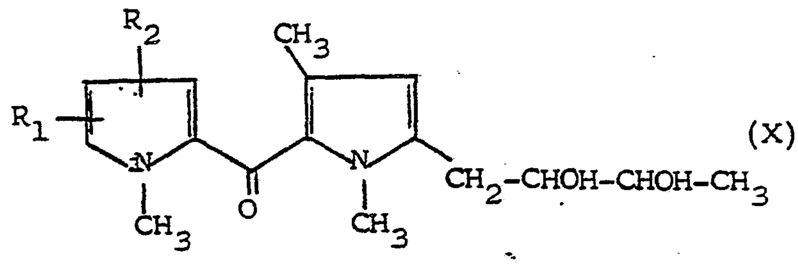

(X)

wherein the variables are as defined in formula (I).

Suitable and preferred values of $R_1$ and $R_2$ are as described under formula (I).

A particularly suitable compound of this invention is therefore 2-carboxymethyl-1,4-dimethyl-5-(1'-methyl-2'-pyrroloyl)pyrrole (3).

A second group of compounds of the present invention of interest are those of the formula (XIII):

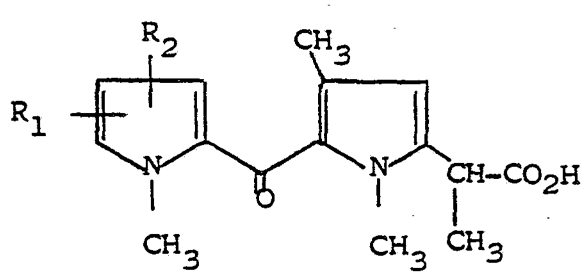

(XI)

wherein $R_1$ and $R_2$ are as defined in formula (I), pharmaceutically acceptable salts thereof and pro-drugs thereof of the formulae (XII), (XIII), (XIV) and (XV):

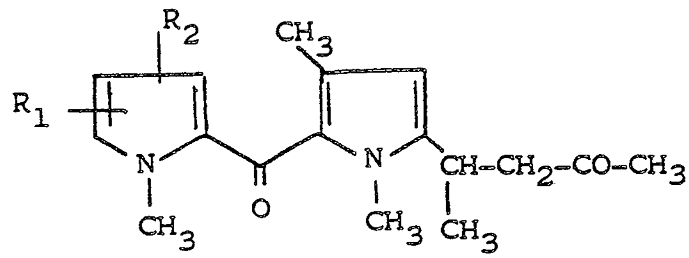

(XII)

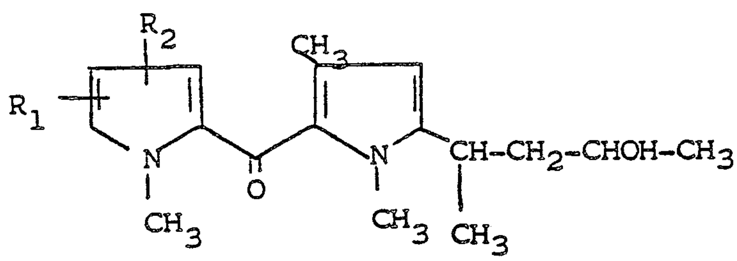

(XIII)

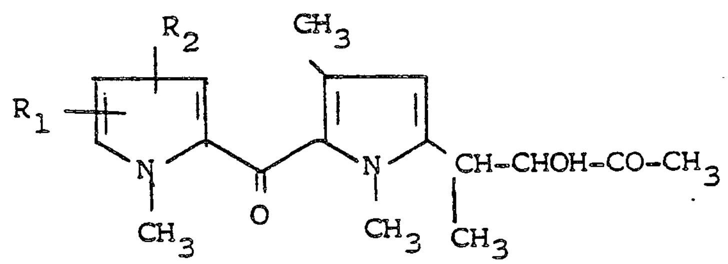

(XIV)

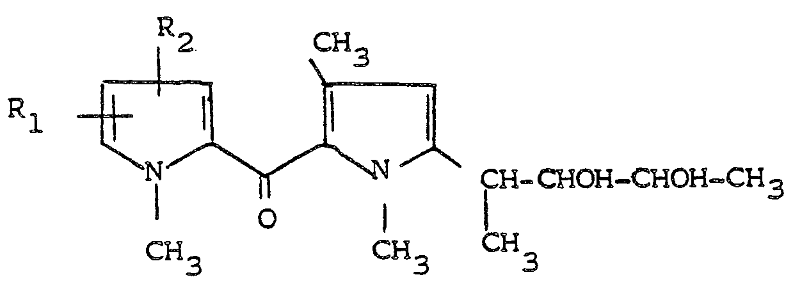

(XV)

A third group of compounds of the present invention of particular interest are those of the formula (XVI):

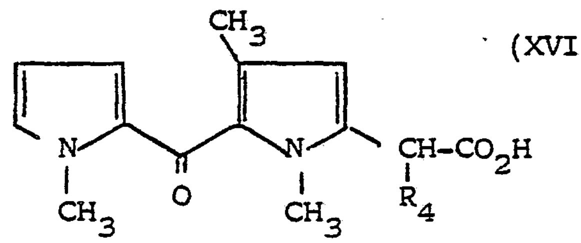

(XVI)

wherein $R_4$ is as defined in formula (I), pharmaceutically acceptable salts thereof and pro-drugs thereof of the formulae (XVII) (XVIII), (XIX) and (XX):

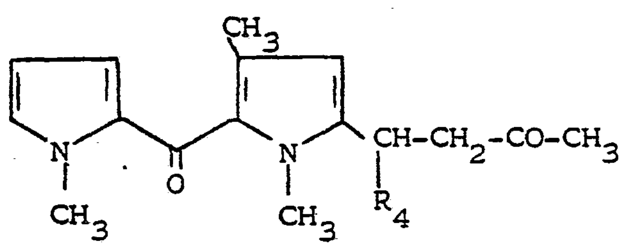

(XVII)

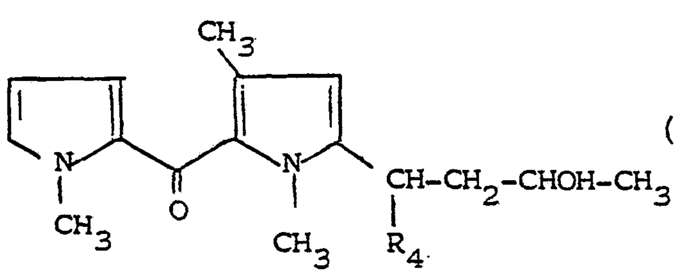

(XVIII)

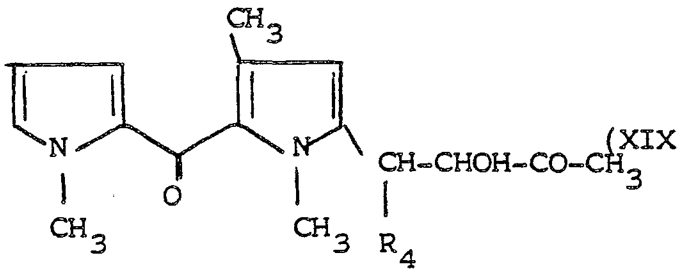

(XIX)

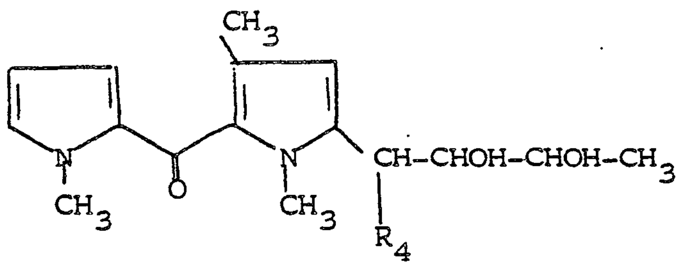

(XX)

wherein $R_4$ is as defined in formula (I):

It will be appreciated that compounds of the formula (I) wherein $R_4$ is methyl and pharmaceutically acceptable salts and pro-drugs thereof have an asymmetric centre. The present invention extends to the enantiomers thereof and the racemates of these enantiomers.

The compounds of the present invention are most suitably provided in crystalline form.

The present invention also provides a process for the preparation of a compound of the formula (I) or a pharmaceutically acceptable salt thereof, which process comprises the basic hydrolysis of a compound of the formula (XXI):

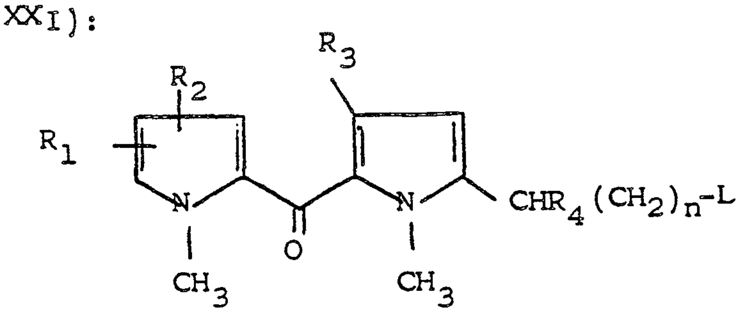

(XXI)

wherein:

$R_1$, $R_2$, $R_3$, $R_4$ and n are as defined in formula (I); and

L is CN or a group $CO_2R_9$ in which $R_9$ is $C_{1-4}$ alkyl;

and thereafter if desired converting $R_3$ when hydrogen in the resultant compound of the formula (I) to $C_{1-6}$ alkyl and if desired acidifying the resulting salt to form a compound of formula (I), or converting the salt to a pharmaceutically acceptable salt thereof, or to another pharmaceutically acceptable salt thereof.

Conversion of $R_3$ when hydrogen to an $R_3$ $C_{1-6}$ alkyl group may be carried out conventionally, for example by sequential reaction with an inorganic base and the relevant alkyl iodide in a polar inert solvent such as tetrahydrofuran. Suitable bases include sodium hydride. A preferred base is the combination of butyllithium and di-isopropylamine.

The hydrolysis may be effected using an hydroxide such as sodium hydroxide, in aqueous ethanol. The free acid of the formula (I) may be prepared by treating the resultant salt with hydrochloric acid or the like. Pharmaceutically acceptable salts of the compounds of the formula (I) may be prepared by ion exchange.

The present invention provides a further process for the preparation of a compound of the formula (I) wherein $R_3$ is $C_{1-6}$ alkyl or a pharmaceutically acceptable salt thereof, which process comprises decarboxylating a compound of the formula (XXIII):

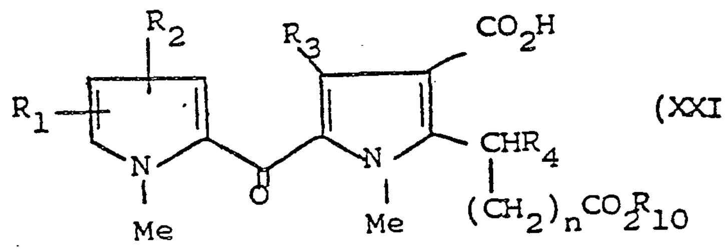

(XXIII)

wherein $R_{10}$ is $C_{1-4}$ alkyl when n = 0 or hydrogen when n = 1 and if desired salifying the resultant compound to give a pharmaceutically acceptable salt directly or converting a salt to a pharmaceutically acceptable salt.

Decarboxylation may be carried out in conventional manner, for instance by heating a melt of the compound of the formula (XXVII)at moderately elevated temperature e.g. 180-220°C, under an inert atmosphere, such as nitrogen, until evolution of carbon dioxide has ceased.

Subsequent optional process steps may be carried out as hereinbefore described.

The following Scheme I shows synthetic pathways to compounds of the formula (I):

Scheme 1

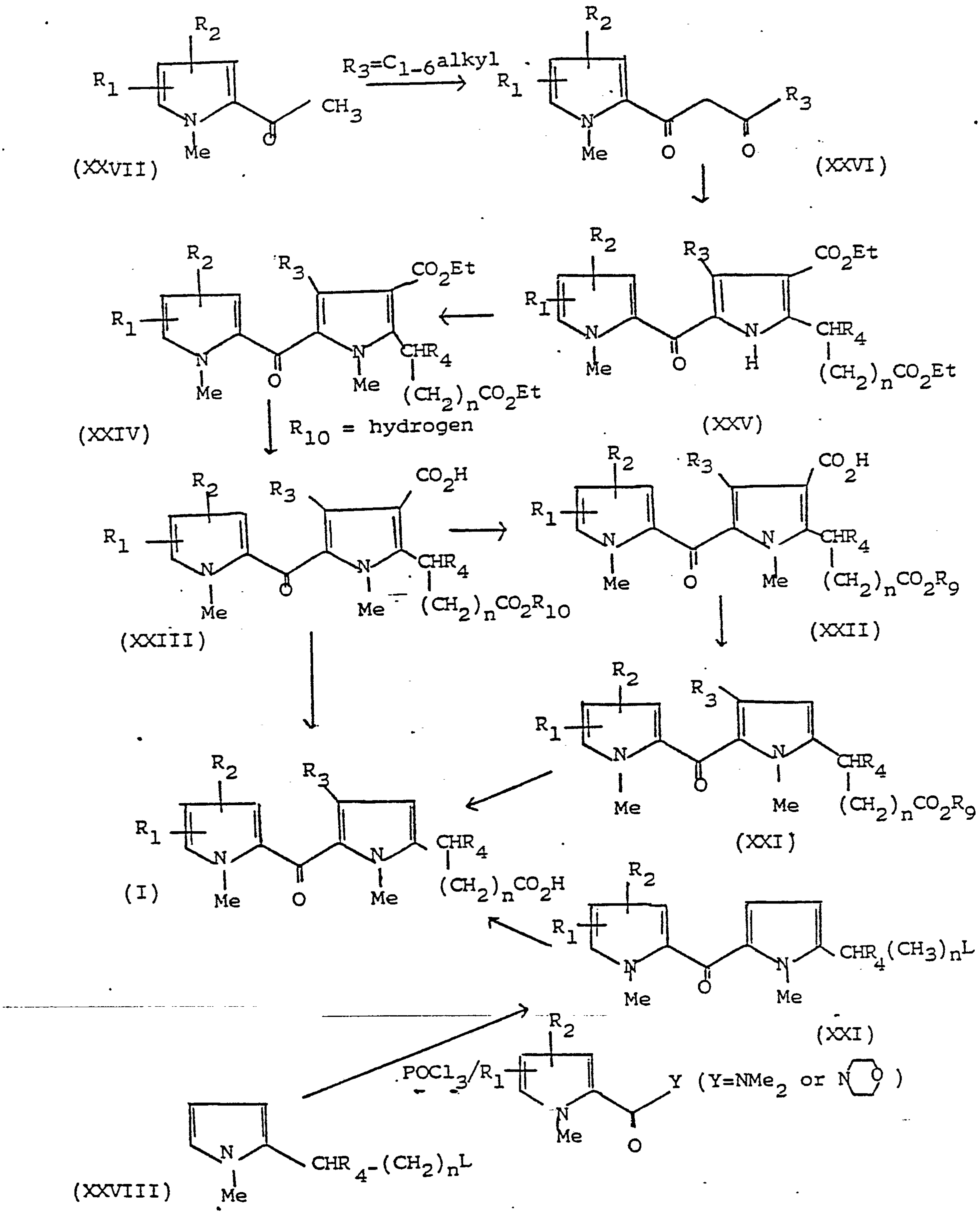


(XXVII) (XXVI) (XXV) (XXIV) (XXIII) (XXII) (XXI) (I) (XXI) (XXVIII)

It is believed that compounds of the formulae (XXI) to (XXVIII) are also novel, and as such intermediates of the formulae (XXI) and (XXIII) form an aspect of the present invention.

Intermediates of the formula (XXI) wherein L = CN may be prepared by treating a compound of the formula (XXVIII) with the complex formed between phosphorus oxychloride and the amide shown in Scheme 1. This reaction is generally carried out under the conditions conventionally used for Vilsmeyer reactions.

Compounds of the formula (XXVIII) are prepared in analogy with known compounds

Intermediates of the formula (XXI) wherein L = $CO_2R_9$ as hereinbefore defined may be prepared by decarboxylation of a compound of the formula (XXII) (see Scheme I), under conditions substantially as hereinbefore described for the decarboxylation of compounds of the formula (XXIII).

Compounds of the formula (XXII) are prepared by analogy with known compounds.

Intermediates of the formula (XXIII) may be prepared by de-esterifying a compound of the formula (XXIV) (see Scheme I), under condition substantially as hereinbefore described for the de-esterification of compounds of the formula (XXI) wherein L = $CO_2R_9$ as defined.

Compounds of the formula (XXIV) may be prepared by analogy with known compounds.

The present invention also provides a process for the preparation of a pro-drug of a compound of the formula (I) which is an in vivo hydrolysable ester thereof, which process comprises esterifying the compound of the formula (I) or active acylating derivative thereof with the corresponding alcohol.

Esterification of the acid itself may be carried out in conventional manner, for instance under acid catalysis and/or under reflux in an inert solvent having a non-extreme boiling point such as less than 100°C. Alternatively it may be carried out in the presence of a dehydrating agent such as dicyclohexylcarbodiimide.

The present invention additionally provides processes for the preparation of a pro-drug of a compound of the formula (I) which is an in vivo hydrolysable amide thereof, which process comprises reacting an active derivative of the compound of the formula (I) with ammonia or a corresponding amine.

Suitable active acylating derivatives of the acid of the formula (I) for esterification and amidation include acid halides such as the acid chloride, the acid anhydride, mixed anhydrides such as those formed from ethyl chloroformate and esters such as the methyl and ethyl esters.

The reactions are normally carried out in non-hydroxylic organic solvent such as tetrahydrofuran, ethyl acetate, toluene, dichloromethane and NN-dimethylformamide. Esterification is normally carried out in the presence of an acid acceptor such as pyridine or triethylamine. The reactions may be carried out at

any non-extreme temperature such as -10°-100°C and more suitably 0-80°C, for amidation most suitably 10-50°C. The higher reaction temperatures are employed with less active derivatives of the acid of the formula (I) such as esters whereas the lower temperatures are employed with the more reactive derivatives of the acid of the formula (I) such as mixed anhydrides.

The present invention further provides a process for the preparation of a pro-drug of a compound of the formula (I) in which the $CO_2H$ group is replaced by a CHO group, which process comprises the reduction of an acid halide of the compound of the formula (I).

The reaction may be suitably carried out under Rosenmund reduction conditions, that is with hydrogen using a palladium/barium sulphate catalyst in an inert organic solvent. A small amount of quinoline and sulphur may be added. Suitable solvents include anhydrous acetone, ethyl acetate and xylene. The reaction may be carried out at a temperature in the range 10 to 150°C.

Tri-n-butyl tin may also be used as a reductant.

The present invention provides a process for the preparation of a pro-drug of a compound of the formula (I) in which the $CO_2H$ group is replaced by a $CH_2OH$ group which process comprises the reduction of a compound of the formula (I) in which the $CO_2H$ group has been replaced by a CHO group.

This reaction may be suitably carried out with a mild complex hydride such as sodium borohydride or a structurally hindered complex hydride such as lithium tri-t-butoxyaluminium hydride. Reaction is usually carried out in a solvent conventionally regarded as compatible with the reductant used

at a non-extreme temperature such as -30 to 80°C, more suitably -10 to 60°C, depending on the reductant employed.

The present invention also provides a process for the preparation of a pro-drug of a compound of the formula (I) wherein the $CO_2H$ group is replaced by a group of the sub-formula (a) as hereinbefore defined, that is, a compound of the formula (II) as hereinbefore defined, which process comprises oxidising a compound of the formula (XXIX)

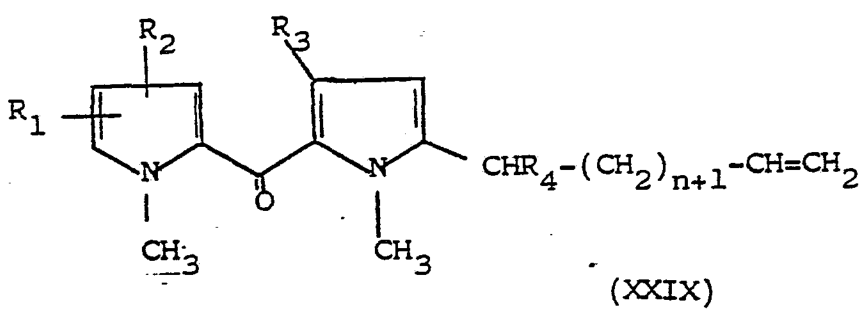

(XXIX)

wherein the variables are as defined in formula (I).

The oxidation is suitably carried out in aqueous dimethylformamide solution in the presence of palladium chloride and cuprous chloride using pure oxygen or air. In general it is sufficient to blow air through the reaction mixture at an ambient or slightly elevated temperature to effect oxidation. The desired compound may be obtained from the reaction mixture by dilution with water followed by extraction into water-immiscible solvent such as chloroform which may then be dried and evaporated. This initial crude material may be purified chromatographically if desired, for example by column chromatography over silica gel using 1:1 ether:petrol as eluant.

The compounds of the formula (XXIX) may be prepared by the decarboxylation of a corresponding compound of the formula (XXX):

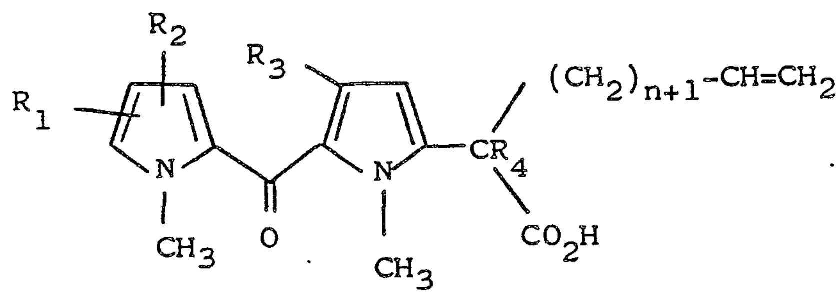

(XXX)

wherein $R_1$, $R_2$, $R_3$ $R_4$ and n are as defined in relation to formula (I).

The decarboxylation may be effected by heating, for example to 170 - 210°C. The desired product may be obtained by trituration under a non-hydroxylic solvent such as chloroform.

The acid of the formula (XXX) may be obtained by hydrolysis of the corresponding ethyl ester using normal sodium hydroxide solution followed by neutralisation with hydrochloric acid.

The desired ethyl ester may be prepared by the alkylation of the corresponding compound of the formula (XXXI):

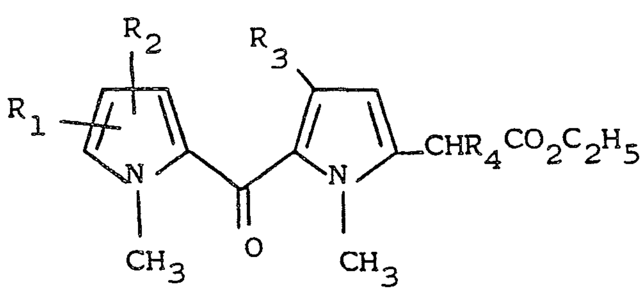

(XXXI)

wherein $R_1$, $R_2$, $R_3$, $R_4$ and n are as defined in relation to formula (I). Such alkylations may be brought about by generating an anion of the formula (XXXI), for example with sodium hydride in dimethoxyethane, and quenching said anion with allyl bromide when n = 0 or 4-bromobut-1-ene when n = 1.

The ethyl ester of a compound of the formula (XXX) wherein $R_4$ is a methyl group may alternatively be prepared by methylation of the corresponding ethyl ester wherein $R_4$ is a hydrogen atom, for example by sequential reaction with sodium hydride and methyl iodide.

Alternatively, compounds of the formula (XXX) may be prepared by direct alkylation of a compound of formula (I) with, for example allyl bromide (when n = 0) in the presence of diisopropylamide.

Compounds of the formula (XXXI) are novel and thus form an aspect of this invention as intermediates to pro-drugs of compounds of the formula (I) of formula (II).

Another process of this invention suitable for preparation of a pro-drug of a compound of the formula (I) wherein the $CO_2H$ group is replaced by a group of the sub-formula (a) as hereinbefore defined comprises the decarboxylation of a compound of formula (XXXII):

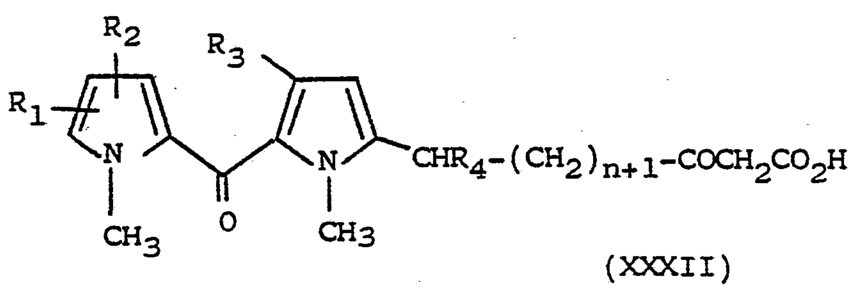

(XXXII)

wherein $R_1$, $R_2$, $R_3$, $R_4$ and n are as hereinbefore defined with relation to formula (I).

The decarboxylation may suitably be carried out by heating in an inert solvent such as DMSO.

Compounds of the formula (XXXII) may be prepared by converting a compound of formula (XXXIII):

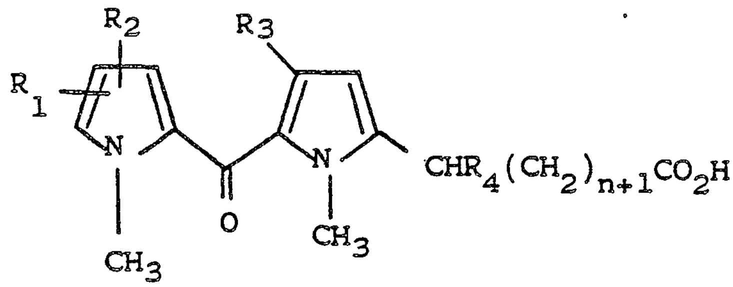

(XXXIII)

to an active derivative thereof such as that wherein the $CO_2H$ group is replaced by $-CO_2COEt$; and then treating the active derivative with a mixture of bis-(trimethylsilyl) malonate and butyllithium (Synthesis 1979 p. 787).

Compounds of the formula (XXXIII) may be prepared as described for compounds of formula (I) or by analogous methods thereto.

The present invention additionally provides a process for the preparation of a pro-drug of a compound of the formula (I) wherein the $CO_2H$ group is replaced by a group of the sub-formula (b) as hereinbefore defined, that is, a compound of the formula (III) as hereinbefore defined, which process comprises the reduction of a corresponding compound of the formula (II) as hereinbefore defined.

Such a reduction may use a complex hydride such as sodium borohydride. Mild conditions and avoidance of excess reagent prevent reduction of the carbonyl adjacent to the pyrrole rings. The desired compound may be purified by conventional methods of column chromatography.

The present invention further provides a process for the preparation of a pro-drug of a compound of the formula (I) wherein the $CO_2H$ group is replaced by a group of the sub-formula (c) as hereinbefore defined, that is a compound of the formula (XXXIV):

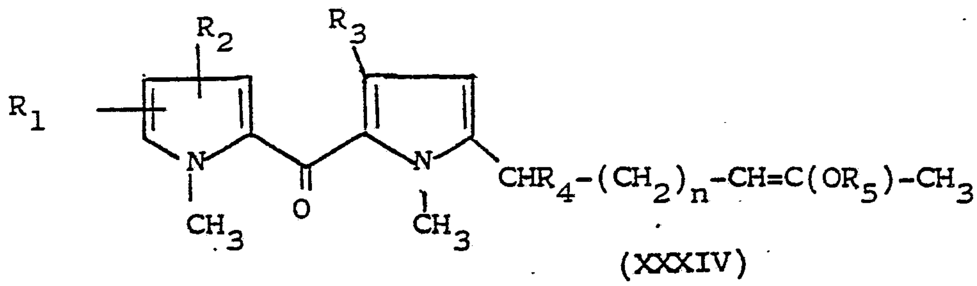

(XXXIV)

wherein $R_5$ is as defined in sub-formula (c) and the remaining variables are as defined in formula (I) which process comprises the enol etherification of a compound of formula (I) wherein the $CO_2H$ group is replaced by a group of sub-formula (a). This process is analogous to that described in Belgian Patent No. 866,857 (US Patent No. 4200645).

The present invention also provides a process for the preparation of a pro-drug of a compound of the formula (I) wherein the $CO_2H$ group is replaced by a group of the sub-formula (d) as hereinbefore defined, that is a compound of the formula (IV) as hereinbefore defined, which process comprises the oxidation of a compound of the formula (XXXIV) as hereinbefore defined.

The oxidation is suitably carried out using m-chloroperbenzoic acid at 0-5°C in mixed solvents such as diethyl ether/water.

The present invention further provides a process for the preparation of a pro-drug of a compound of the formula (I) wherein the $CO_2H$ group is replaced by a group of the sub-formula (e) as hereinbefore defined that is a compound of the formula (V) as hereinbefore defined, which process comprises the reduction of a corresponding compound of the formula (IV) as hereinbefore defined.

Such a reduction may be effected using a complex hydride such as sodium borohydride under conditions which are conventional therefor.

The present invention additionally provides processes for the preparation of pro-drugs of compounds of the formula (I) wherein the $CO_2H$ group is replaced by a group of the sub-formulae (f), (g), (h), (i) or (j) as hereinbefore defined, that is, compounds of the formulae (XXXV), (XXXVI), (XXXVII), (XXXVIII) or (XXXIX):

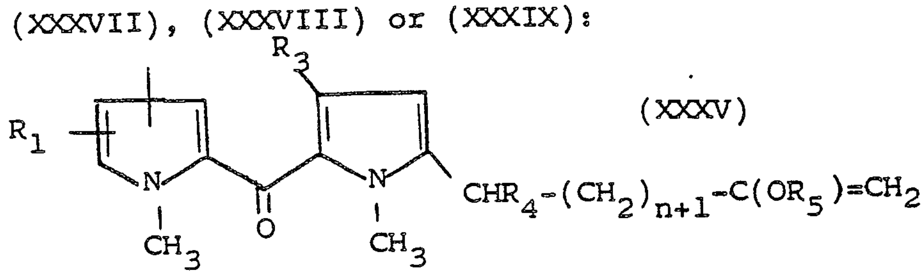

(XXXV)

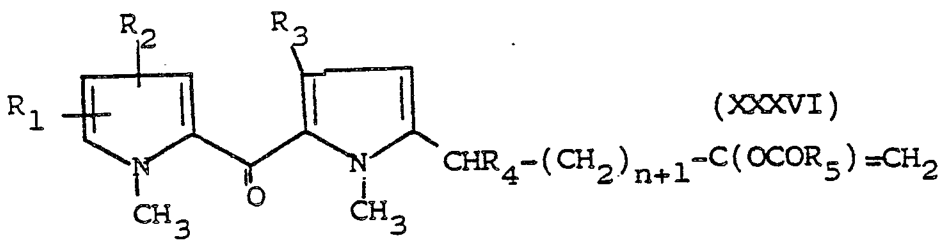

(XXXVI)

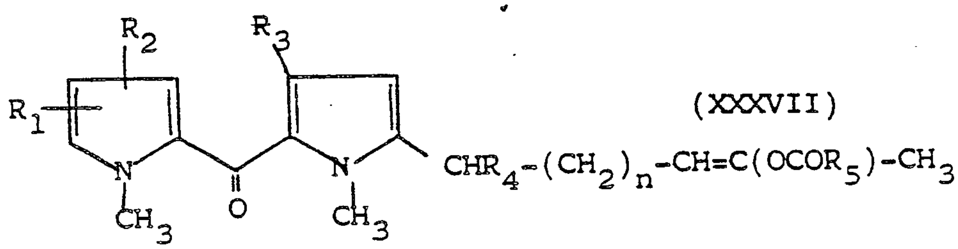

(XXXVII)

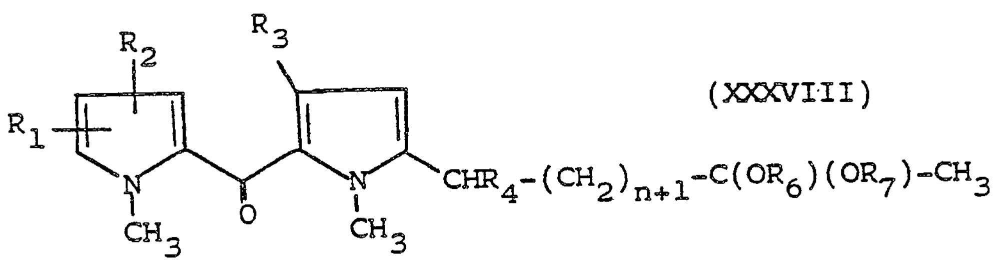

(XXXVIII)

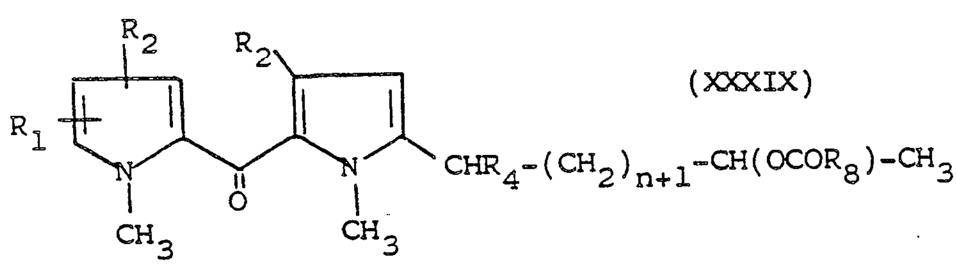

(XXXIX)

wherein $R_5$, $R_6$, $R_7$ and $R_8$ are as defined in sub-formulae (a) - (k) and the remaining variables are as

defined in formula (I). These processes are as conventional, and are analogous to those described in Belgium Patent No. 866,857 (US Patent No. 4200645); more specifically, enol acylation, enol etherification and acetal formation may be carried out as described in US Patent No. 4180585; and acylation may be carried out as described in UK Patent No. 1538473.

Scheme II

(I)

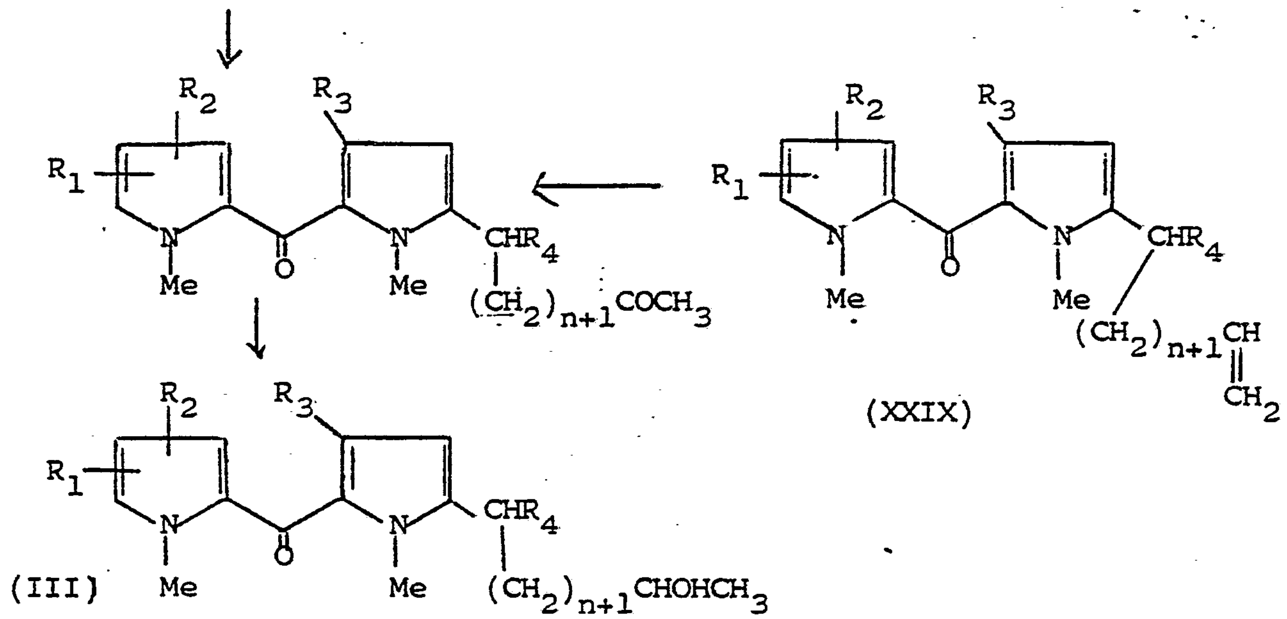

(XXIX)

(III)

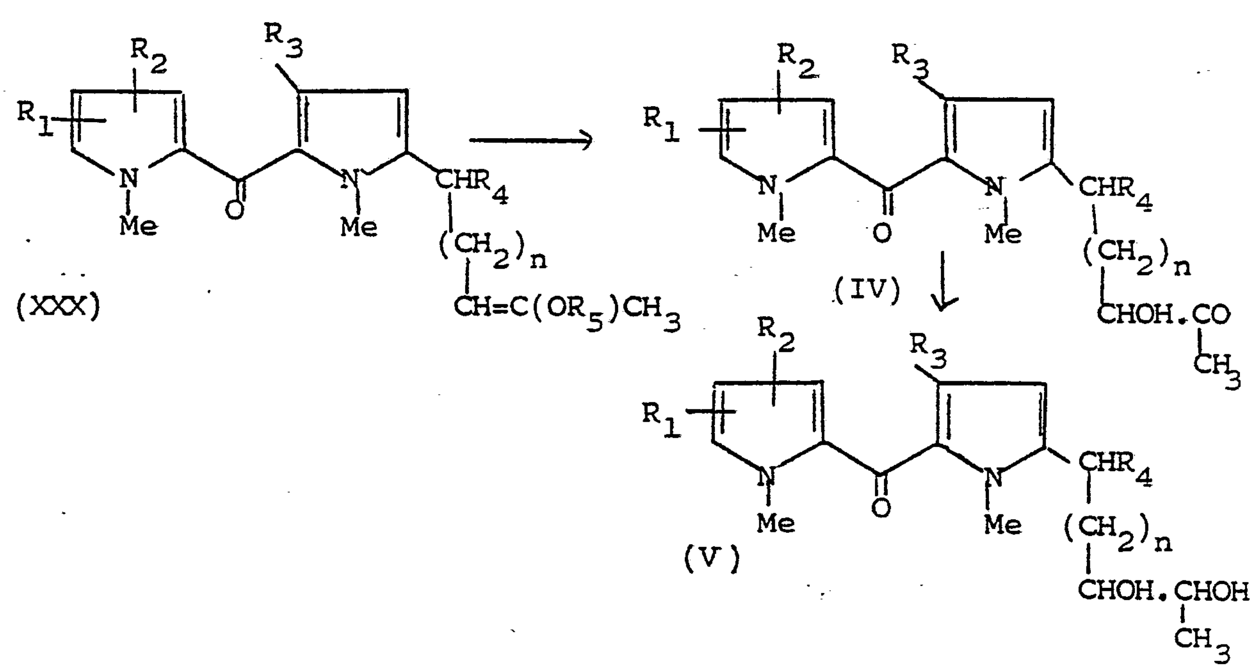

(XXX)

(IV)

(V)

The enantiomers of compounds of the formula (I) wherein $R_4$ is methyl and pharmaceutically acceptable salts and pro-drugs thereof may be resolved from their racemates by conventional resolution techniques.

In a further aspect this invention provides a pharmaceutical composition which comprises a compound of the formula (I) or a pharmaceutically acceptable salt or pro-drug thereof, and a pharmaceutically acceptable carrier.

The compositions of this invention are useful in treating rheumatic and arthritic conditions because of their anti-inflammatory and analgesic properties. The compositions may be adapted for administration via the topical, oral, rectal or injection routes but it is preferred that they are adapted for oral administration.

The compositions of this invention may contain diluents, binders, fillers, disintegrants, flavouring agents, colouring agents, lubricants, preservatives or the like in conventional manner. These conventional excipients may be employed in conventional manner, for example as in the preparation of compositions of ketoprofen, indomethacin, naproxen, acetylsalicylic acid or other anti-inflammatory analgesic agents.

Most suitably the composition of this invention will be in the form of a unit dose such as a tablet, capsule or reconstitutable powder in a sachet. Such unit doses will generally contain from 10 mg to 1000 mg and more suitably will contain from about 30 mg to 500 mg for example 50 mg to 250 mg of active agent, for example about 50, 100, 150, 200, 250, 300, 350, 400, 450 or 500 mg. These compositions may be administered once or more times a day, for example 2, 3 or 4 times daily, so that the total daily dose for a 70

kg adult will usually be in the range of 100 to 3000.mg and more usually in the range 300 to 3000 mg for example 500 to 2000 mg. Alternatively the unit dose may contain from 2-20 mg of active agent and may be administered in multiples if desired to give the preceeding daily dose.

A favoured form of the composition of this invention is a hard gelatin capsule containing the active agent. The active agent may be in the form of a powder, granulate or the like and may advantageously be in intimate mixture with a lubricant such as magnesium stearate.

A further favoured form of the composition of this invention is a tablet containing the active agent. The active agent may be in the form of a recompressed granulate of the active ingredient in intimate mixture with a lubricant such as magnesium stearate, a filler such as microcrystalline cellulose and a disintegrant such as sodium starch glycollate.

This present invention also provides a method of treating inflammatory and/or painful conditions in mammals which comprises administering per day from 50 to 4000 mg of a compound of this invention and more usuallly from 100 to 3000 mg for example from 200 to 1500 of a compound of this invention.

Mammals which may be thus treated include humans and domestic animals such as dogs, cats or horses.

Most suitably the medicament will be administered orally as 2, 3 or 4 doses per day at the dose level previously indicated.

Description 1

2-Cyanomethyl-1-methyl-5-(1'-methyl-2'-pyrroloyl)pyrrole (d.1)

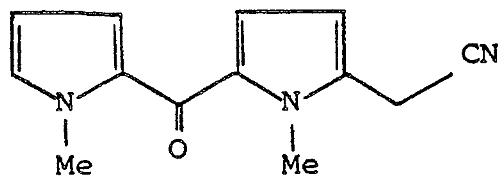

(d.1)

Oxalyl chloride (15 ml, 0.17 mole) was added to 1-methylpyrrole-2-carboxylic acid (10 g, 0.08 mole) in dry toluene (100 ml) and the mixture refluxed for 2½ hr. After allowing to cool the solvent was evaporated off and the residue dissolved in dry diethyl ether (100 ml) before dropwise addition with cooling of dimethylamine (15 ml, 0.23 mole) in dry diethyl ether. After completion of the addition the solution was stirred for 1½ hr. and then the precipitated dimethylamine hydrochloride was filtered off and the filtrate evaporated to give the required dimethylamide.

The dimethylamide (12.16 g, 0.08 mole) was treated with phosphoryl chloride (9.3 ml, 0.1 mole) under nitrogen and the mixture stirred at 55°C for 1 hr. 1-Methyl-pyrrole-2-acetonitrile (18 g, 0.15 mole) was added to the resulting complex and the mixture stirred for 6½ hr. at 120°C. After cooling the resulting solid was dissolved in water (200 ml), basified with dilute aqueous sodium hydroxide and stirred for ½ hr. at 60°C. The mixture was again allowed to cool and after acidifying was extracted with chloroform (150 ml). The extracts were dried ($MgSO_4$), the solvent removed under vacuum and the residue chromatographed (silica gel [400 g] eluted with ether) to give

the required product.

N.m.r., δ ($CCl_4$): 4.78 (3H, m); 4.1 (2H, m); 3.96 (3H, s); 3.89 (3H, s); 3.69 (2H, s).

Example 1

2-Carboxymethyl-1-methyl-5-(1'-methyl-2'-pyrroloyl)pyrrole (1)

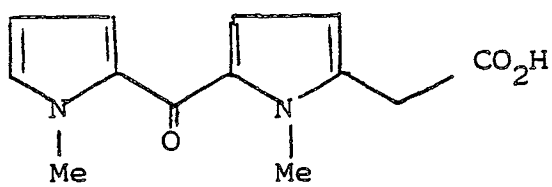

(1)

The above acetonitrile (d.1.) (330 mg, 0.00145 mole) was refluxed for 3 hr. in a solution of dilute aqueous sodium hydroxide/methanol (50 ml). On cooling the solution was washed with diethyl ether (2 x 25 ml) and the aqueous layer acidified with conc. HCl. The resulting precipitate was extracted into chloroform (2 x 50 ml) and the organic layer separated, dried ($MgSO_4$) and evaporated to dryness. The residue was decolourised with charcoal and recrystallised from diethyl e ther to give the required acid.

M.pt: 155-157°C

N.m.r., δ ($CDCl_3$): 10.00 (1H, brs); 6.80 (3H, m); 6.11 (2H, m); 3.93 (3H, s); 3,86 (3H, s); and 3.72 (2H, s).

Analysis: Found: C, 63.41; H, 5.77; N, 11.38
$C_{13}H_{14}N_2O_3$ requires: C, 63.42; H, 5.69; N, 11.38.

Description 2

4-(1'-Methyl-2'-pyrryl)butane-2,4-dione (d.2)

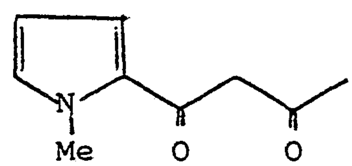

(d.2)

A solution of 2-acetyl-1-methylpyrrole (44.3 g, 0.36 mole) in dry dimethoxyethane (250 ml) was added dropwise to a cooled, stirred solution of sodium hydride (21.6 g of 80% dispersion in oil, 0.72 mole) and ethyl acetate (70 ml, 0.72 mole) in dimethoxyethane (150 ml) under nitrogen. After the hydrogen evolution had ceased, the solution was heated under reflux for 2½ hr. On cooling, the reaction mixture was treated with a few drops of water before being acified with aqueous 5N HCl. The brown oil which resulted was extracted into diethyl ether (2 x 150 ml) and then washed with 10% aqueous NaOH (2 x 100 ml).

The combined aqueous washings were acidified with conc. HCl solution and then extracted into diethyl ether (2 x 100 ml). The organic layer was washed with water (2 x 100 ml), dried ($Na_2SO_4$) and concentrated to leave 4-(1'methyl-2'-pyrryl)butane-2,4-dione as a brown oil (51.5 g, 87%).

Description 3

3-Ethoxycarbonyl-2-ethoxycarbonylmethyl-4-methyl-5-(1'methyl-2'-pyrroloyl)pyrrole (d.3)

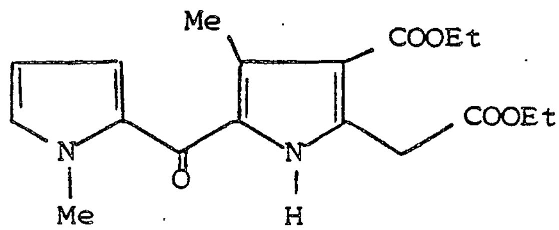

(d.3)

A solution of sodium nitrite (8.97 g, 0.13 mole) in water (13 ml) was added dropwise to a mixture of the above diketone (d.2 ) (20.8 g, 0.126 mole) in glacial acetic acid (200 ml), the temperature being maintained just below 8°C. After completing the addition the mixture was stirred at the same temperature for a further ½ hr. and allowed to come to room temperature over 2 hr. The resulting solution was then added dropwise to a solution of diethyl acetone 1,3-dicarboxylate (22.9 ml, 0.126 mole) in glacial acetic acid (200 ml) at 70°C. At the same time there was added portionwise a mixture of zinc (26.4 g, 0.378 mole) and sodium acetate (31 g, 0.378 mole), the temperature being kept below 90°C. After complete addition, the mixture was refluxed for 1 hr. and then poured slowly into water (3 l). The black oil produced was extracted into ethyl acetate, and the extract then washed with 10% aqueous NaOH and water before being dried ($Na_2SO_4$) and concentrated to leave a black oily solid (37 g). This material was filtered through a silica column (300 g) using diethyl ether eluant and the solid obtained was recrystallised from toluene/60-80° petrol

to afford the diester as a pink solid.

M.pt: 102-5°C

N.m.r., δ ($CCl_4$): 11.27 (1H, brs); 6.8-6.4 (2H, m); 5.98 (1H, m); 4.16 (2H, q, J~7Hz); 5.06 (2H, q, J ~ 7Hz); 3.87 (5H, s); 2.34 (3H, s); 1.29 (3H, t, J ~ 7Hz); and 1.24 (3H, t, J ~ 7Hz).

<u>3-Methoxycarbonyl-2-(2'-methoxycarbonylethyl)-4-methyl-5-(1"-methyl-2"-pyrroloyl)pyrrole</u> (d.4) was prepared analogously using dimethyl 3-oxo-adipate in place of diethyl acetone-1,3-dicarboxylate (24% yield, white solid).

M.pt: 109-110°C

N.m.r., δ ($CCl_4$): 10.90 (1H, br, s); 6.90-6.35 (2H, m); 6.10-5.80 (1H, m); 3.83 (3H, s); 3.70 (3H, s); 3.57 (3H, s); 3.13 (2H, d, J = 7Hz); 2.57 (2H, d, J = 7Hz) and 2.30 (3H, s).

Description 4

3-Ethoxycarbonyl-2-ethoxycarbonylmethyl-1,4-dimethyl-5-(1'-methyl-2'-pyrroloyl)pyrrole (d.5)

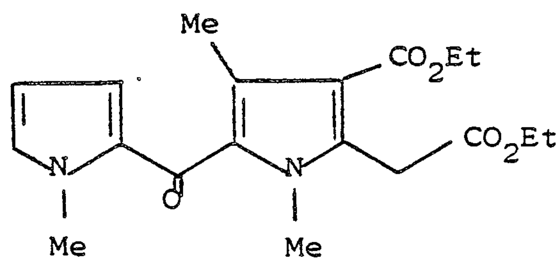

(d.5)

Anhydrous potassium carbonate (17.9 g, 0.13 mole) followed by dimethyl sulphate (10.4 ml, 0.11 mole) was added to a solution of the above diethyl ester (d.3) (18 g, 0.052 mole) in ethyl methyl ketone (300 ml), and the mixture was refluxed for 16 hr. After cooling the solution was poured into water (400 ml), and the product extracted into diethyl ether (2 x 150 ml). The ether layers were washed with water (2 x 100 ml), dried ($Na_2SO_4$) and evaporated to dryness. The resulting solid was recrystallised from diethyl ether/petrol to afford the diester as a pink solid.

M.pt.: 94-95°C

N.m.r., δ ($CDCl_3$): 6.85 (1H, m); 6.63 (1H, m); 6.10 (1H, m); 4.28 (2H, q, J ~ 7Hz); 4.18 (2H, q, J ~ 7Hz); 4.10 (2H, s); 3.96 (3H, s); 3.56 (3H, s); 2.20 (3H, s); 1.33 (3H, t, J ~ 7Hz); and 1.26 (3H, t, J ~ 7Hz).

<u>3-Methoxycarbonyl-2-(2'-methoxycarbonylethyl)-1,4-dimethyl-5-(1"-methyl-2"-pyrroloyl)pyrrole</u> (d.6) was prepared analogously from the diester (d.4). (91% yield, white solid).

M.pt.: 97-98$^{\circ}$C

N.m.r., δ ($CDCl_3$): 6.95-6.75 (1H, m); 6.75-6.50 (1H, m); 6.27-6.02 (1H, m); 3.97 (3H, s); 3.78 (3H, s); 3.66 (3H, s); 3.57 (3H, s); 3.50 - 3.0 (2H, m); 2.90-2.40 (2H, m) and 2.17 (3H, s).

Description 5

3-Carboxy-2-carboxymethyl-1',4-dimethyl-5-(1'-methyl-2'-pyrroloyl)pyrrole (d.7)

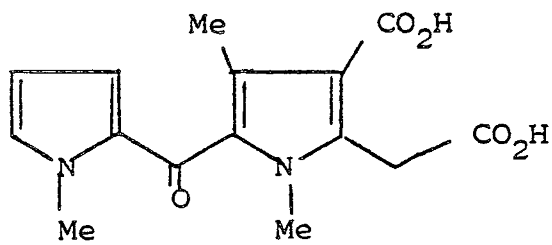

(d.7)

The above N-methylated diester (d.5) (12.93 g, 0.036 mole) in ethanol (50 ml) and 25% sodium hydroxide solution (200 ml) were refluxed for 1½ hr. After cooling the solution was poured into dilute hydrochloric acid (400 ml) with cooling and stirring. The resulting white precipitate was filtered off, washed with water and dried to afford the required diacid.

M.pt: 236-240°C (dec.)

N.m.r., δ ($CDCl_3/d_6$ DMSO): 6.97 (1H, m); 6.57 (1H, m); 6.08 (1H, m); 4.10 (2H, s); 3.94 (3H, s); 3.52 (3H, s); and 2.14 (3H, s).

<u>3-Carboxy-2-(2'-carboxyethyl)-1,4-dimethyl-5-(1"-methyl-2"-pyrroloyl)pyrrole</u> (d.8) was prepared analogously from the diester (d.6) (98% yield, white solid).

M.pt.: 197-198$^{o}$C

N.m.r, δ ($d_6$DMSO): 11.93 (2H, br, s); 7.20 (1H, t, J = 2Hz); 6.64 (1H, dd, J = 4 and 2Hz); 6.14 (1H, dd, J = 4 and 2Hz); 3.95 (3H, s); 3.60 (3H, s); 3.2 -2.2 (4H, m) and 2.15 (3H, s).

Description 6

3-Carboxy-2-ethoxycarbonylmethyl-1,4-dimethyl-5-(1'-methyl-2'-pyrroloyl)pyrrole (d.9)

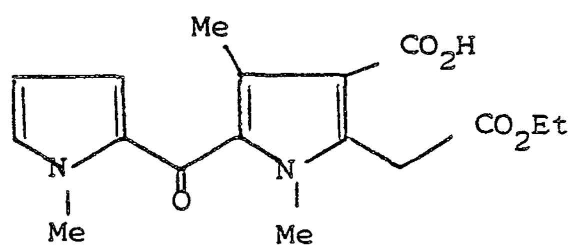

(d.9)

The above diacid (d.7) (3.5 g, 0.0115 mole) was suspended in 0.5% ethanolic hydrochloric acid (45 ml) and refluxed for $1^{1}/_{4}$ hr. by which time all the solid had dissolved. The precipitate which formed on cooling the solution was collected, washed with ethanol and dried to afford the ester as a white solid.

N.m.r., δ ($CDCl_3$): 6.87 (1H, m); 6.68 (1H, m); 6.10 (1H, m); 4.19 (2H, q, J ~ 7Hz); 4.13 (2H, s); 3.57 (3H,s); 2.21 (3H, s); and 1.27 (3H, t, J ~ 7Hz).

Example 2

2-Ethoxycarbonylmethyl-1,4-dimethyl-5-(1'-methyl-2'-pyrroloyl)pyrrole (d.10)

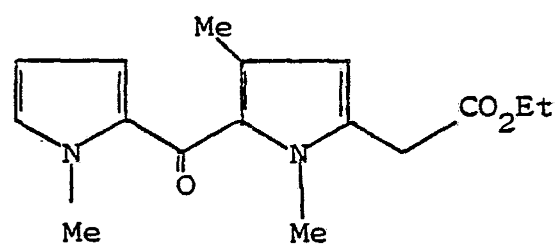

(2)

The above monoester (d.9) (3.46 g, 0.0104 mole) was heated under nitrogen to 210°C for 2½ hr. After cooling the resulting brown oil was dissolved in chloroform (100 ml) washed with water (50 ml), dilute aqueous sodium hydroxide (50 ml) and finally water (2 x 50 ml). The organic layer was dried ($Na_2SO_4$), and evaporated to dryness and the resulting oil purified by column chromatography using silica gel (60 g) eluted with 1:1 ether:petrol to give the required product.

N.m.r., δ ($CDCl_3$): 6.77 (1H, m); 6.61 (1H, m); 6.05 (1H, m); 5.85 (1H, s); 4.14 (2H, q, J ~ 7Hz); 3.9 (3H, s); 3.58 (5H, s); 2.00 (3H, s); and 1.25 (3H, t, J ~ 7Hz).

Example 3

2-Carboxymethyl-1,4-dimethyl-5-(1'-methyl-2'-pyrroloyl) pyrrole (2)

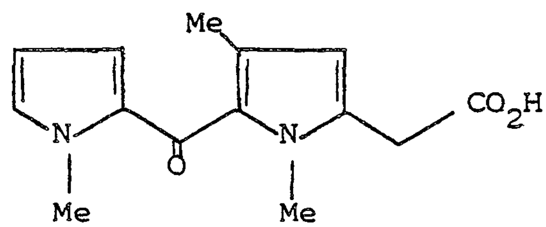

(3)

2-Ethoxycarbonylmethyl-1,4-dimethyl-5-(1-methyl-2-pyrroloyl)pyrrole (2) (2.7 g, 0.0094 mole) was refluxed in ethanol (25 ml) and 25% aqueous sodium hydroxide (100 ml) for 2 hr. After cooling the solution was acidified with concentrated hydrochloric acid and the cloudy precipitate extracted into chloroform (100 ml). The organic layer was washed with water, dried ($Na_2SO_4$) and evaporated to dryness to give an orange oil which solidified after triturating with 60-80$^{o}$ petrol. The solid was recrystallised from ether/chloroform with charcoal decolourisation to afford the acid as a pale yellow solid.

M.pt: 119-120$^{o}$C

N.m.r., δ ($CDCl_3$): 9.73 (1H, brs); 6.79 (1H, m); 6.63 (1H, m); 6.08 (1H, m); 5.88 (1H, s); 3.92 (3H, s); 3.63 and 3.60 (5H, overlapping s); and 2.00 (3H, s).

Example 4

2-(2'-Carboxyethyl)-1,4-dimethyl-5-(1"-methyl-2"-pyrroloyl) pyrrole (4)

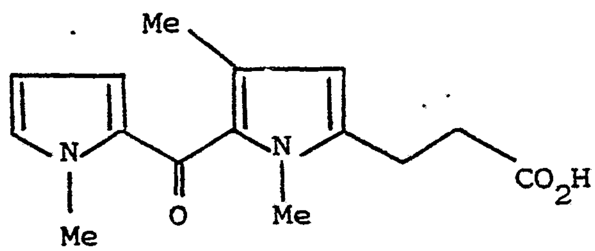

(4)

3-Carboxy-2-(2'-carboxyethyl)-1,4-dimethyl-5-(1"-methyl-2"-pyrroloyl)pyrrole (d.8) (8.35 g, 0.0264 mole) was heated at about 200-210°C under nitrogen for 1½ hr. The residue solid was recrystallised twice from diethyl ether/chloroform using decolourising charcoal to afford the title compound as a white solid (6.31 g, 87%).

| | |
|---|---|
| M.pt.: | 147-149°C |
| N.m.r., δ ($CDCl_3$): | 10.83 (1H, s); 6.77 (1H, t, J = 2Hz); 6.60 (1H, dd, J = 4 and 2Hz); 6.03 (1H, dd, J = 4 and 2Hz); 5.73 (1H, s), 3.90 (3H, s); 3.60 (3H, s); 3.0-2.6 (4H, m) and 1.97 (3H, s). |

Analysis: Calculated for $C_{15}H_{18}N_2O_3$: C, 65.69; H, 6.57; N, 10.22%.

Found: C, 65.50; H, 6.65; N, 10.03%.

DESCRIPTION 7

4[1', 4''-Dimethyl-5'-(1''-methyl-2-''-pyrroloyl 1]-2'-pyrryl]but-1-ene

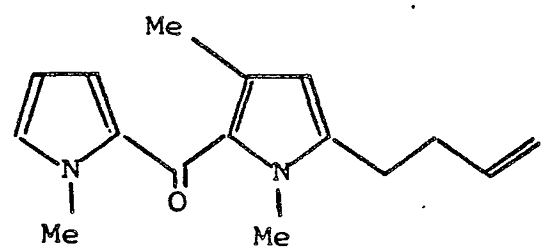

(d7)

The acetic acid of Example 3 (3.01g, 0.0116 mole) was taken up in dry THF (50ml) and added dropwise with cooling to a solution of di-isopropylamine (3.4ml, 0.024 mole) and butyllithium (14.5ml of 1.6M sol., 0.0232ml) in dry THF (30 ml) under nitrogen.

The solution formed was then allowed to come to room temperature over 2 hours before allyl bromide (1ml, 0.0116 mole) was added. The reaction mixture was left stirring at room temperature for 2½ hours.

On cooling in an ice bath, water (20 ml) was added to the solution and was then acidified with dil. HCl acid. The product was then extracted into ether, washed with water, dried and evaporated down to leave a dark solid.

The crude product (2.2 g) was heated under nitrogen at about 200°C for 45 mins. The solid melted at about 120°C and decarboxylation was seen to occur almost immediately.

On cooling, the solid remaining was taken up in chloroform, washed with water, dried and concentrated to leave a dark solid.

The resulting solid was chromatographed on silica (100g) using 25% ether/petrol as eluent to afford a pale yellow oil which crystallized on cooling.

Recrystallisation from 40-80 petrol gave the title compound as a white solid (48-50°C).

Analysis

| $C_{16}H_{21}N_2O$: | | Calc. | Found |
|---|---|---|---|
| | C | 69.77 | 69.95 |
| | H | 6.98 | 7.29 |
| | N | 10.85 | 10.88 |

Example 5

4-(1',4'-Dimethyl-5'-(1"-methyl-2"-pyrroloyl)-2'-pyrryl) butan-2-one

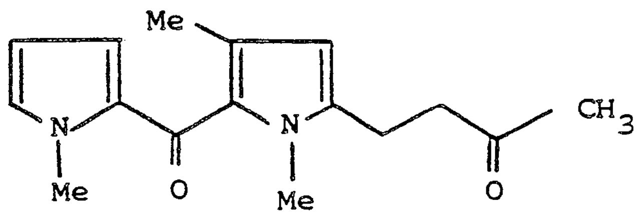

(5)

2-(2'-Carboxyethyl)-1,4-dimethyl-5-(1"-methyl-2"-pyrroloyl)pyrrole from Example 4 (11.36 g, 0.041 mole) was dissolved in dry tetrahydrofuran (100 ml) together with dry triethylamine (5.8 ml, 0.041 mole) at -20°C under nitrogen. Ethyl chloroformate (4.0 ml, 0.41 mole) was added dropwise and solution stirred at -20°C for 1 hour before storing at the same temperature for 24 hours prior to use. The white precipitate was filtered off and the filtrate added at 0°C to a solution prepared by the treatment of bis-(trimethylsilyl)malonate (20.0 g, 0.091 mole) in dry ether (150 ml) under nitrogen at -65°C with a solution of n-butyllithium in hexane (46.7 ml, 1.95 M; 0.091 mole). The resulting solution was stirred at 0°C for 15 minutes before quenching the reaction by addition of 5% aqueous sodium bicarbonate (200 ml). The aqueous layer was separated and the organic layer further extracted with sodium bicarbonate solution (2 x 100 ml) before combining the aqueous layers and acidifying with dilute hydrochloric acid. The acidic solution was extracted with ethyl acetate (2 x 150 ml), the organic layers then combined, washed with water (2 x 100 ml) and solvent evaporated off to leave a red oil. This was

heated at 80°C in dimethyl sulphoxide (50 ml) for 20 minutes until gas evolution ceased. The solution was poured into saturated aqueous sodium bicarbonate (200 ml) and the resulting solution extracted with ether (2 x 150 ml). The organic layers were combined, washed with water (2 x 80 ml), dried (anhydrous $Na_2SO_4$) and concentrated to give a pale yellow solid. This was purified by recrystallisation from 1:1 hexane:ether to give the title product as a white, crystalline solid, m.p. 110-111°C, n.m.r. $\delta$ ($CDCl_3$) 6.78 (1H, t, J=2Hz), 6.60 (1H, dd, J=4 and 2Hz), 6.08 (1H, dd, J=4 and 2Hz), 5.68 (1H, s), 3.91 (3H, s), 3.60 (3H, s), 2.80 (4H, s), 2.17 (3H, s) and 1.97 (3H, s).

The title compound may also be prepared by oxidation of the butene (d7) in aqueous dimethylformamide, with palladium chloride in the presence of air.

Example 6

4-(1',4'-Dimethyl-5'-(1"-methyl-2"-pyrroloyl)-2'-pyrryl) butan-2-ol

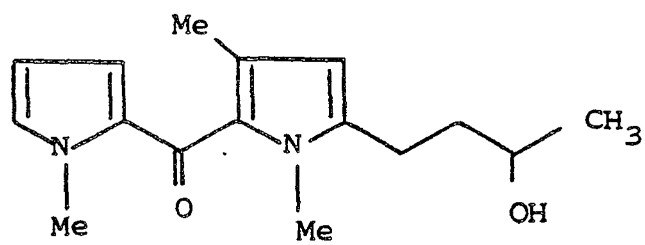

The butanone of Example 5 (2.0 g, 0.0074 mole) was dissolved in ethanol (100 ml) and stirred for 1 hour at room temperature with sodium borohydride (0.27 g, 0.0074 mole). After the addition of saturated ammonium chloride solution (30 ml) the mixture was concentrated and the residue partitioned between ether (100 ml) and water (100 ml). The ether layer was washed with water (2 x 30 ml) dried (anhydrous $Na_2SO_4$) and concentrated. The resulting solid was recrystallised from 1:1 petrol:ether to yield the title compound as a white crystalline solid, m.p. 78-79°C, n.m.r. $\delta$ ($CDCl_3$) 6.70 (1H, t, J=2Hz), 6.53 (1H, dd, J=4 and 2Hz), 5.97 (1H, dd, J=4 and 2Hz), 5.65 (1H, s), 4.0-3.3 (1H, m), 3.85 (3H, s), 3.55 (3H, s), 2.78 (1H, s), 2.7-2.4 (2H, m), 1.95 (3H, s), 1.9-1.6 (2H, m) and 1.18 (3H, d, J=6Hz).

Example 7

2-Acetoxy-4-(1',4'-Dimethyl-5'-(1"-methyl-2"-pyrroloyl)-2'-pyrryl)butane

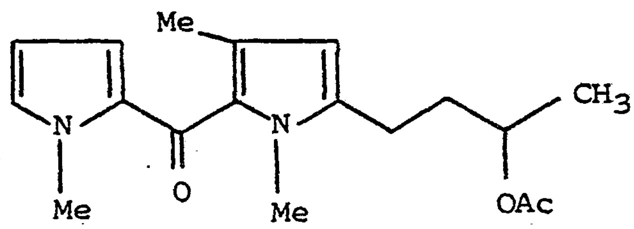

The butanol of Example 6 (1.8 g, 0.0066 mole) was dissolved in dry toluene (50 ml) together with pyridine (3 ml) and cooled in an ice bath. The solution was treated dropwise with acetyl chloride (1.5 ml, 0.02 mole) and stirred at room temperature for 2 hours. The solution was poured into ice/water (100 ml) and then extracted with ether (2 x 80 ml). The organic layer was washed with water, 1M HCl, water again, dried (anhydrous $Na_2SO_4$) and concentrated. The crude product was chromatographed on silica gel using ether as eluant and the resulting oil was crystallised from 2:1 60-80$^o$ petrol:ether to give the title compound as a white, crystalline solid, m.p. 34-35$^o$C, n.m.r. ($CDCl_3$) 6.73 (1H, t, J=2Hz), 6.57 (1H, dd, J=4 and 2Hz), 6.02 (1H, dd, J=4 and 2Hz), 5.68 (1H, s), 4.92 (1H, sextet, J=6Hz), 3.88 (3H, s), 3.57 (3H, s), 2.7-2.4 (2H, m), 2.0-1.7 (2H, m), 2.02 (3H, s), 1.98 (3H, s) and 1.27 (3H, d, J=6Hz).

EXAMPLE 8

2-(2'-Ethoxycarbonylethyl)-1-methyl-5(1''-methyl-2''-pyrroloyl) pyrrole (8)

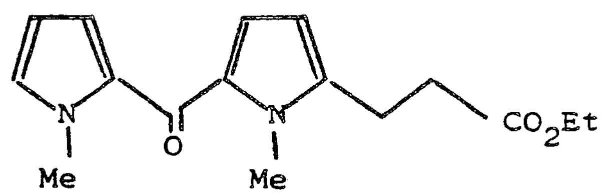

(8)

In a similar manner to that carried out in Description 1, the title compound was prepared by the reaction of the appropriate morpholino amide and 2-(2'-ethoxycarbonylethyl)-1-methyl-pyrrole.

EXAMPLE 9

4-(2'-Carboxyethyl)-1-methyl-5-(1''-methyl-2''-pyrroloyl)pyrrole

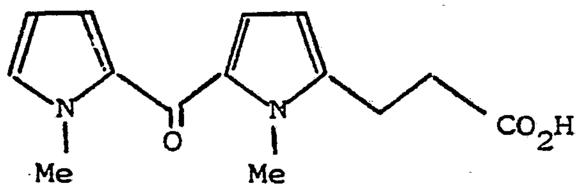

(9)

In a similar manner to Example 8 the title acid was prepared from the corresponding ethyl ester (8).

EXAMPLE 10

4-(1′-Methyl-5′-(1″-methyl-2″-pyrroloyl)-2′-pyrryl butan-2-one (10)

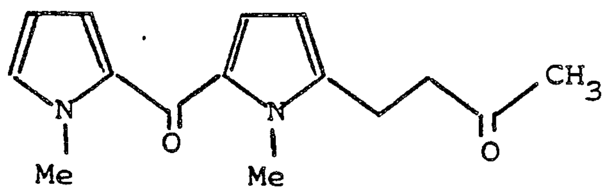

(10)

In a similar manner to that carried out in Example 5, the title compound was prepared from the acid (9)

mp 64-65°C

Analysis for $C_{16}H_{18}N_2O_2$

| | Calc. | Found |
|---|---|---|
| C | 69.77 | 69.95 |
| H | 6.98 | 7.29 |
| N | 10.85 | 10.88 |

PHARMACOLOGICAL DATA

1. Analgesic activity

a. Mouse writhing test

In a conventional phenylquinone induced mouse writhing test, compound 1, 3 and 4 were active at the following dose per os.

% inhibition

| Compound | 16 mg/kg |
|---|---|
| 1 | 90 |
| 3 | 100 |
| 4 | 60 |

The $ED_{50}$ value for compound 3 was calculated in a separate test to be 2.8 mg/kg po.

2. Anti-inflammatory activity

a. Carrageenin test

In a conventional carageenin induced oedema test in the rat, the results were as follows:

| Compound | Dose (mg/kg) | Mean Oedema | % inhibition | |
|---|---|---|---|---|
| Control | -- | 145.3 + 10.3 | -- | |
| 1 | 20 | 117.7 + 6.6 | 19 | * |
| Control | -- | 106.0 + 6.01 | -- | |
| 3 | 12.5 | 70.62 + 6.31 | 33 | ** |

Compounds 5, 6 and 7 produced inhibitions which were significantly different from controls.

b. Cotton Pellet test

In a convention cotton pellet induced granuloma test, the results were as follows:

| Compound | Dose (mg/kg) | Granuloma Wt. (mg) | % inhibition |
|---|---|---|---|
| Control | -- | 48.1 | -- |
| 1 | 10 | 37.6 | 22 * |
| | | | |
| Control | -- | 35.2 | -- |
| 3 | 5 | 26.1 | 26 ** |

* $0.05>p>0.02$ (Significance as assessed by Students 't' test)
** $0.01>p>0.001$

Toxicity

No toxic effects were observed in any of the above tests.

Claims

1. A compound of formula (I):

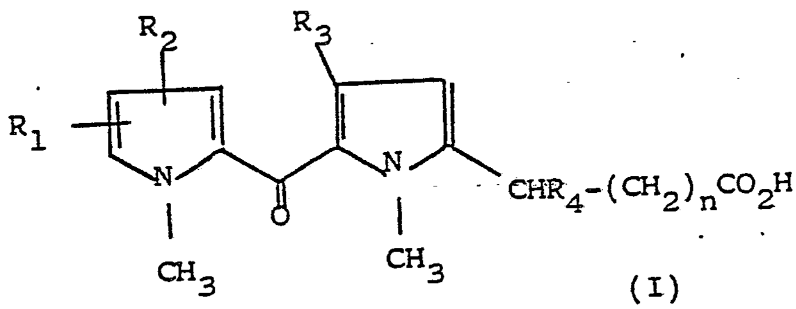

(I)

or a pharmaceutically acceptable salt or a pro-drug thereof, wherein:

$R_1$ and $R_2$ are the same or different and are hydrogen, halogen or $C_{1-4}$ alkyl;

$R_3$ is hydrogen or $C_{1-6}$ alkyl

$R_4$ is hydrogen or methyl; and

n is 0 or 1.

2. A compound according to claim 1 wherein the moiety of formula:

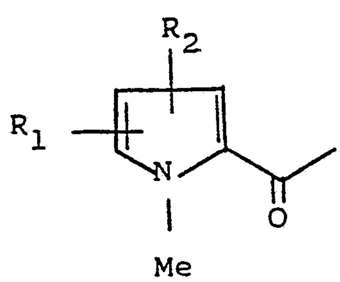

is:

4-chloropyrroloyl, 4-bromopyrroloyl, 4,5-dichloropyrroloyl, 4,5-dibromopyrroloyl, 5-methyl-

pyrroloyl, 5-ethylpyrroloyl, 3,5-dimethylpyrroloyl, 3,5-diethylpyrroloyl, 4,5-dimethylpyrroloyl, or 4,5-diethylpyrroloyl.

3. A compound according to claim 1 wherein $R_1$ and $R_2$ are both hydrogen.

4. A compound according to any one of the claims 1 to 3 wherein $R_3$ is methyl.

5. A compound according to any one of the claims 1 to 4 wherein n is 0.

6. A pro-drug of a compound according to claim 1 wherein the $CO_2H$ group is replaced by an in vivo hydrolysable ester or amide group, a CHO or $CH_2OH$ group or wherein the $CO_2H$ group is replaced by a group of one of the sub-formulae (a) - (j):

| | |
|---|---|
| $-CH_2-CO-CH_3$ | (a) |
| $-CH_2-CHOH-CH_3$ | (b) |
| $-CH=C(OR_5)-CH_3$ | (c) |
| $-CHOH-CO-CH_3$ | (d) |
| $-CHOH-CHOH-CH_3$ | (e) |
| $-CH_2-C(OR_5)=CH_2$ | (f) |
| $-CH_2-C(OCOR_5)=CH_2$ | (g) |
| $-CH=C(OCOR_5)-CH_3$ | (h) |
| $-CH_2-C(OR_6)(OR_7)-CH_3$ | (i) |
| $-CH_2-CH(OCOR_8)-CH_3$ | (j) |

wherein:

$R_5$ is $C_{1-4}$ alkyl;

$R_6$ and $R_7$ are each $C_{1-4}$ alkyl or together are $-CH_2CH_2-$ or $-CH_2CH_2CH_2-$; and

$R_8$ is $C_{1-4}$ alkyl optionally substituted by phenyl or optionally salified amino.

7. A compound according to claim 1 of formula (VI)

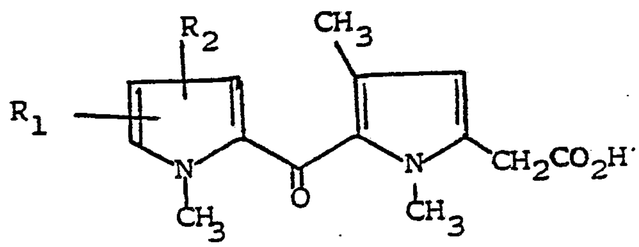

(VI)

or a pharmaceutically acceptable salt thereof or a pro-drug thereof of formula (VIII), (IX), (X), (XI) or (XII)

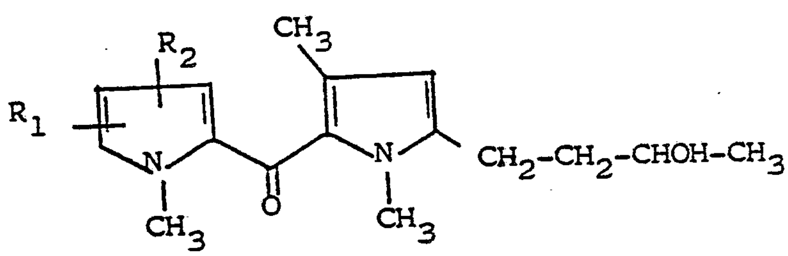

(VIII)

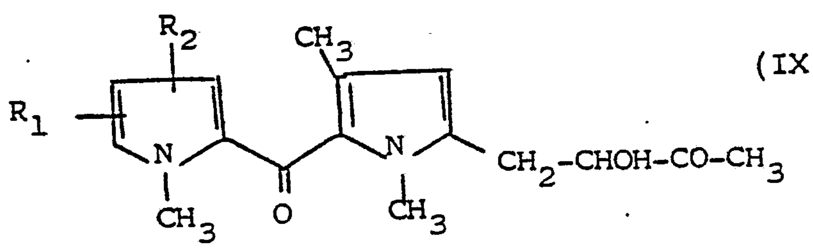

(IX)

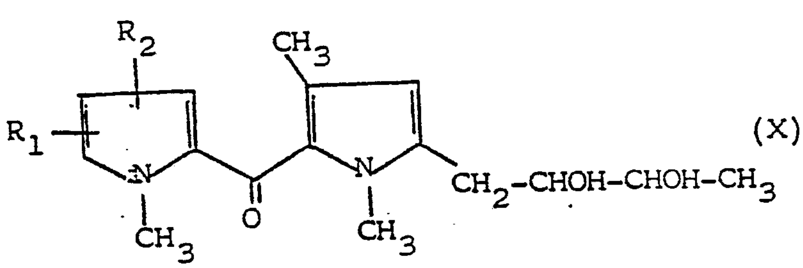

(X)

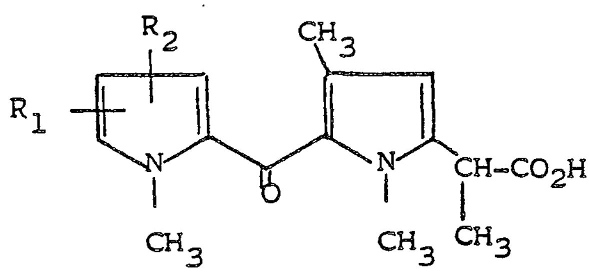

(XI)

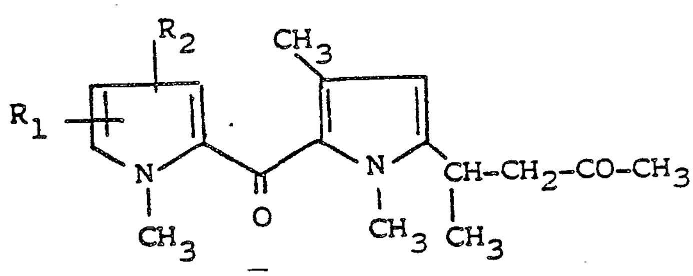

(XII)

wherein the variables are as defined in claim 1.

8. 2-Carboxymethyl-1-methyl-5-(1'-methyl-2'-pyrroloyl)pyrrole or 2-ethoxycarbonylmethyl-1,4-dimethyl-5-(1'-methyl-2'-pyrroloyl)pyrrole.

9. A process for the preparation of a compound according to claim 1, or a pharmaceutically acceptable salt thereof characterised by either:

i) the basic hydrolysis of a compound of the formula (XXI):

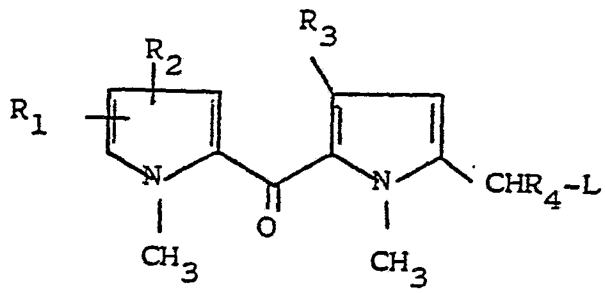

(XXI)

wherein:

$R_1$, $R_2$, $R_3$ and $R_4$ are as defined in claim 1 and L is CN or a group $CO_2R_9$ in which $R_9$ is $C_{1-4}$ alkyl and therafter if desired converting $R_3$ when hydrogen in the resultant compound of the formula (I) to $C_{1-6}$ alkyl and if desired acidifying the resulting salt to form a compound of formula (I), or converting a salt to a pharmaceutically acceptable salt thereof, or to another pharmaceutically acceptable salt thereof; or

ii) when $R_3$ is $C_{1-6}$ alkyl, the decarboxylation of a compound of the formula (XXIII):

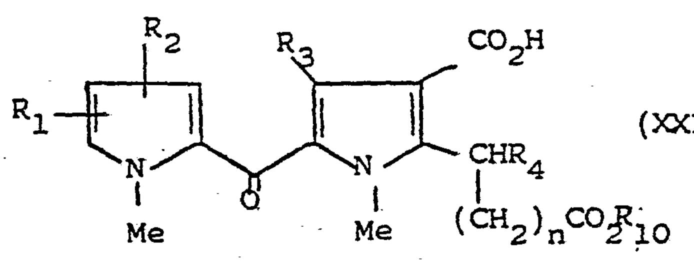

(XXIII)

wherein $R_{10}$ is $C_{1-4}$ alkyl when n is 0 or hydrogen when n is 1; and therafter if desired salifying the resultant compound to give a pharmaceutically acceptable salt or converting a salt to a pharmaceutically acceptable salt.

10. A process for the preparation of a pro-drug

according to claim 6 characterised by, when the COOH group is replaced by:

i) an *in vivo* hydrolysable ester group; esterifying a compound according to claim 1 or an active acylating derivative with the corresponding alcohol;

ii) an *in vivo* hydrolysable amide group, reacting an active derivative of a compound according to claim 1 with ammonia or a corresponding amine;

iii) an CHO group or $CH_2OH$ group reducing an acid halide of a compound according to claim 1 and thereafter if desired further reducing the resulting compound wherein $CO_2H$ is replaced by CHO;

iv) a group of sub-formula (a) as defined in claim 6, oxidising a compound of the formula (XXIX):

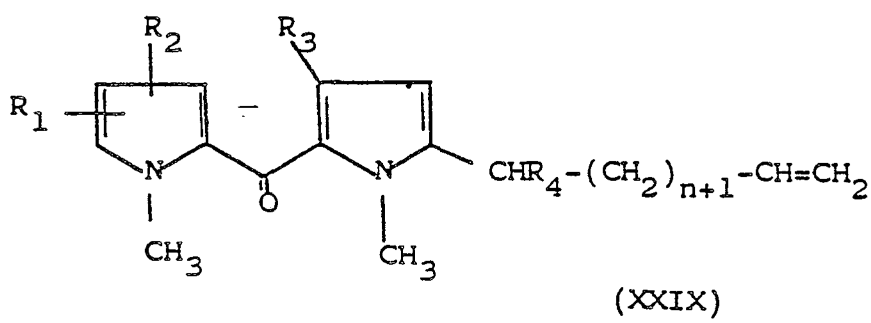

(XXIX)

or decarboxylating a compound of formula (XXXII):

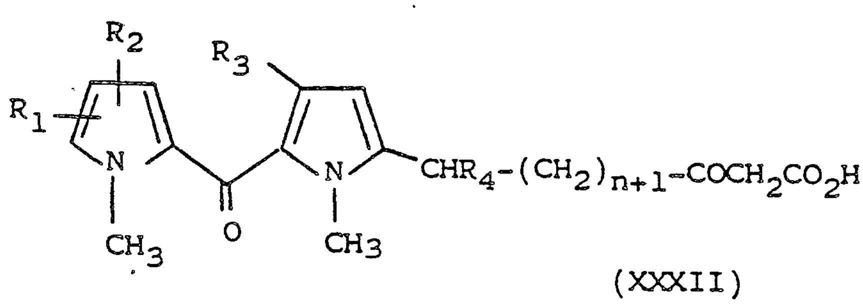

(XXXII)

wherein the variables are as defined in claim 1;

v) a group of sub-formula (b) as defined in claim 6, reducing a compound according to claim 1 wherein the $CO_2H$ group is replaced by a group of sub-formula (a);

vi) a group of sub-formula (c) as defined in claim 6, enol etherifying a compound according to claim 1 wherein the $CO_2H$ group is replaced by a group of sub-formula (a);

vii) a group of sub-formula (d) as defined in claim 6, oxidising a compound according to claim 1 wherein the $CO_2H$ group is replaced by a group of sub-formula (c);

viii) a group of sub-formula (e) as defined in claim 6, reducing a compound according to claim 1 wherein the $CO_2H$ group is replaced by a group of sub-formula (d);

viv) a group of sub-formula (f), (g), (h), (i) or (j) as defined in claim 6, a process as described in Belgium Patent No. 866,587.

11. A pharmaceutical composition comprising a compound according to any one of claims 1 to 9 or a pharmaceutically acceptable salt or a pro-drug thereof and a pharmaceutically acceptable carrier.

12. A compound according to any one of the claims 1 to 9 or a pharmaceutically acceptable salt or a pro-drug thereof for use as in treating inflammatory and/or painful conditions in mammals.

European Patent Office

**PARTIAL EUROPEAN SEARCH REPORT**
which under Rule 45 of the European Patent Convention shall be considered, for the purposes of subsequent proceedings, as the European search report

0032048

Application number
EP 80304709.1

## DOCUMENTS CONSIDERED TO BE RELEVANT

| Category | Citation of document with indication, where appropriate, of relevant passages | Relevant to claim | CLASSIFICATION OF THE APPLICATION (Int Cl ) |
|---|---|---|---|
| | EP - A1 - 0 001 534 (ROLLAND)<br>+ Abstract; claim 1 + | 1,6,12 | C 07 D 207/333<br>C 07 D 207/337 |
| P | &. US-A-4 194 003 (LAFOREST) (18-03-1980)<br>-- | | A 61 K 31/40 |
| | US - A - 4 132 788 (WONG)<br>+ Column 1, lines 20-68 +<br>---- | 1,7,11 | |

TECHNICAL FIELDS SEARCHED (Int. Cl )

C 07 D 207/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 6-10
Claims searched incompletely: 1-5, 11, 12
Claims not searched: -
Reason for the limitation of the search:

In the claims 1-5, 11 and 12 there is no explanation of the term "pro-drug" given.

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| VIENNA | 07-04-1981 | ONDER |